# EUROPÄISCHE PATENTANMELDUNG

(11) **EP 3 811 909 A1**
(43) Veröffentlichungstag der Anmeldung: **28.04.2021**
(21) Anmeldenummer: 19204988.0
(22) Anmeldetag: 24.10.2019
(51) Int. Cl.: A61F 5/01, A61F 13/06

(54) **TEXTILTEIL**

(71) Anmelder: medi GmbH & Co. KG, 95448 Bayreuth (DE)
(72) Erfinder: Bauer, Patrick, 91275 Auerbach (DE)
(74) Vertreter: Lindner Blaumeier Patent- und Rechtsanwälte

(57) **Zusammenfassung**

Textilteil, insbesondere textiles orthopädisches Hilfsmittel, umfassend einen textilen Grundkörper (2) sowie wenigstens ein am Grundkörper (2) angeordnetes Funktionselement (4) aus Kunststoff, wobei wenigstens ein das auf dem Grundkörper (2) angeordnete Funktionselement (4) nur abschnittsweise übergreifendes, flächiges Abdeckelement (5) vorgesehen ist, das sich auch über den Grundkörper (2) erstreckt, wobei das Abdeckelement (5) mit dem Grundkörper (2) und/oder dem Funktionselement (4) verbunden ist und das Funktionselement (4) mit dem Grundkörper (2) stoffschlüssig verbunden ist.

## Beschreibung

Die Erfindung betrifft ein Textilteil, insbesondere textiles orthopädisches Hilfsmittel, umfassend einen textilen Grundkörper sowie wenigstens ein am Grundkörper angeordnetes Funktionselement aus Kunststoff.

Ein solches Textilteil, wie beispielsweise aus EP 2 779 963 B1 bekannt, ist beispielsweise als Manschette oder als Bandage ausgeführt und dient der Anlage am Körper eines Trägers. Das Textilteil weist einen textilen Grundkörper auf, an dem wenigstens ein Funktionselement aus Kunststoff angeordnet ist, das beispielsweise als längliche Schiene oder als Gelenk oder Ähnliches ausgeführt ist. Dieses Funktionselement dient dazu, dem Textilteil eine bestimmte funktionale Eigenschaft zu verleihen, die dem Träger hilfreich ist, also beispielsweise stabilisierend oder versteifend wirkt, um so gegen mechanische Einwirkungen auf die von dem Textilteil übergriffene Körperpartie zu schützen und ähnliches.

Neben der Möglichkeit, ein solches Funktionselement in eine bevorzugt stricktechnisch an dem Grundkörper ausgebildete Tasche einzubringen, also beispielsweise einen Stabilisierungsstab in eine entsprechende längliche Tasche einzuschieben, ist es auch bekannt, das Funktionselement über eine stoffschlüssige Verbindung, also eine Schweißverbindung, am Grundkörper zu fixieren. Dies ist beispielsweise aus EP 2 779 963 B1 bekannt, wo ein Funktionselement über ein mit Kunststoff kaschiertes Trägerelement auf den Grundkörper, beispielsweise eine Bandage, aufgeschweißt wird. Das Funktionselement selbst ist auf das Trägerelement aufgebracht, was in einem ersten Prozessschritt beispielsweise durch Aufspritzen oder Hinterspritzen erfolgt. Dieses Kombinationsbauteil wird sodann in einem zweiten Prozessschritt auf die Bandage aufgelegt und ausschließlich im Bereich des Trägerelements, das breiter als das Funktionselement ist, aufgeschweißt, was durch Aufschmelzen der Kunststoffbeschichtung des Trägerelements erfolgt. Bei dieser Ausgestaltung ist also das Funktionselement selbst nicht mit dem Grundkörper verbunden, sondern lediglich über das Trägerelement, was dazu führt, dass es zu Einbußen in der Funktionalität des Funktionselements kommen kann, da dessen Eigenschaften, beispielsweise stabilisierende Eigenschaften, nicht unmittelbar auf den Grundkörper übertragen werden. Darüber hinaus ist auch der Herstellvorgang aufwendig, da zunächst das Kombinationsbauteil aus Funktionselement und Trägerelement in einem ersten Prozessschritt hergestellt werden muss, wonach erst in einem zweiten Prozessschritt die Applikation dieses Kombinationsbauteils am Grundkörper erfolgt.

Der Erfindung liegt damit das Problem zugrunde, ein demgegenüber verbessertes Textilteil anzugeben.

Zur Lösung dieses Problems ist bei einem Textilteil der eingangs genannten Art erfindungsgemäß vorgesehen, dass wenigstens ein das auf dem Grundkörper angeordnete Funktionselement nur abschnittsweise übergreifendes flächiges Abdeckelement vorgesehen ist, das sich auch über den Grundkörper erstreckt, wobei das Abdeckelement mit dem Grundkörper und/oder dem Funktionselement verbunden ist und das Funktionselement mit dem Grundkörper stoffschlüssig verbunden ist.

Bei dem erfindungsgemäßen Textilteil ist ein sehr einfacher Aufbau vorgesehen, der es ermöglicht, dass einerseits das Funktionselement seine funktionalen Eigenschaften bestmöglich auf den Grundkörper übertragen kann, andererseits aber auch eine sichere Fixierung des Funktionselements am Grundkörper wie auch eine entsprechende teilweise Abdeckung des Funktionselements, die das Funktionselement schützt und kaschiert, gegeben ist. Erfindungsgemäß wird das Funktionselement unmittelbar stoffschlüssig mit dem Grundkörper verbunden, was über eine entsprechende Schweißverbindung erfolgt, indem das aus Kunststoff bestehende Funktionselement lokal an der Grenzfläche zum textilen Grundkörper aufgeschmolzen wird, sodass der aufgeschmolzene Kunststoff das textile Gestrick benetzt, in dieses gegebenenfalls geringfügig eindringt und sich beim Aushärten fest mit diesem verbindet. Dies gewährleistet einen optimalen Funktionsübergang vom Funktionselement auf den Gestrickkörper, wie auch eine bestmögliche Fixierung. Darüber hinaus ist ein das Funktionselement nur abschnittsweise übergreifendes, flächiges und bevorzugt flexibles Abdeckelement vorgesehen, das sich auch über den Grundkörper erstreckt, und das entweder am Grundkörper, am Funktionselement oder an beiden fixiert ist. Dieses Abdeckelement kaschiert das Funktionselement sowie dessen Befestigungsebene zum Grundkörper, schützt diese also gegen eine etwaige Beschädigung, was für die Haltbarkeit der stoffschlüssigen Verbindung von Vorteil ist.

Gegenüber dem bekannten Stand der Technik, wie insbesondere EP 2 779 963 B1 zeichnet sich das erfindungsgemäße Textilteil also durch einen sehr einfachen Aufbau auf, da auf ein Trägerelement verzichtet wird, nachdem das Funktionselement unmittelbar am textilen Grundkörper angeschweißt wird. Es ist demzufolge kein aufwändiger Herstellschritt für ein entsprechendes Kombinationsbauteil erforderlich, vielmehr erfolgt eine direkte Applikation des Funktionselements am Grundkörper. Aufgrund dieser direkten Anbringung ist ein optimaler Funktionsübertrag zum Grundkörper und damit auch zum Träger des Textilteils möglich, wie auch über das Abdeckelement eine versteckte Anordnung der Befestigungsebene des Funktionselements gewährleistet ist.

Grundsätzlich sind damit erfindungsgemäß drei zu unterscheidende Ausgestaltungen denkbar. Gemäß einer ersten Erfindungsalternative kann das Abdeckelement mit dem Grundkörper und dem Funktionselement verbunden sein. Hier sind also quasi drei Befestigungsebenen gegeben, nämlich einerseits die Verbindung des den Grundkörper übergreifenden Abdeckelements mit dem Grundkörper und dem Funktionselement, zum anderen die stoffschlüssige Verbindung des Funktionselements mit dem Grundkörper. Das Abdeckelement ist folglich zweifach fixiert. Ist, wie besonders bevorzugt vorgesehen, das Abdeckelement mit einem entsprechenden Kunststoffanteil ausgerüstet, also beispielsweise mit einer Kunststoffkaschierung, so kann auch die Verbindung des Abdeckelements zum Grundkörper stoffschlüssig sein, das heißt, dass sowohl die Verbindung zum Funktionselement als auch die zum Grundkörper stoffschlüssig ist, was den besonderen Vorteil mit sich bringt, als alle drei Verbindungen in einem einzigen Prozessschritt erwirkt werden können, indem die Bereiche am Abdeckelement und am Funktionselement durch Energiezufuhr aufgeschmolzen werden, so dass es zu einer stoffschlüssigen Schmelzverbindung zwischen Abdeckelement und Grundkörper sowie Funktionselement einerseits und Funktionselement und Grundkörper andererseits kommt.

Eine zweite Alternative sieht vor, dass das Abdeckelement nur mit dem Funktionselement verbunden ist und über den Grundkörper lose liegt. Bei dieser Erfindungsausgestaltung liegen die Befestigungsbereiche oder -ebenen von Abdeckelement zum Funktionselement und vom Funktionselement zum Abdeckelement quasi, gesehen von dem Grundkörper, hintereinander. Das Abdeckelement ist in den Bereichen, wo es das Funktionselement übergreift, entweder vollumfänglich respektive vollflächig oder nur lokal mit dem Funktionselement verbunden. Diese Verbindung kann besonders bevorzugt ebenfalls stoffschlüssig sein, da wie beschrieben das Funktionselement aus Kunststoff ist und demzufolge entsprechend durch Energiezufuhr aufgeschmolzen werden kann, so dass es zu einer stoffschlüssigen Verbindung zum Abdeckelement kommt, gleich wie dieses ausgeführt ist respektive aus welchem Material es ist, solange dieses eine entsprechende Benetzung und damit stoffschlüssige Verbindung gewährleistet. Natürlich besteht auch die Möglichkeit, das Abdeckelement selbst mit einem entsprechenden Kunststoffanteil auszurüsten, beispielsweise mit einer Kunststoffkaschierung, so dass es durch Energieeintrag selbst auch lokal aufgeschmolzen werden kann, um die stoffschlüssige Verbindung zu realisieren. Quasi unterhalb dieser ersten Verbindungsebene ist dann die zweite Verbindungsebene, in der das Funktionselement stoffschlüssig mit dem Grundkörper verbunden ist. Auch hier besteht also die Möglichkeit, beide Verbindungen in einem einzigen Prozessschritt durch eine einmalige Energiezufuhr durch Aufschmelzen der entsprechenden Bauteile in den relevanten Bereichen auszubilden.

Eine dritte Erfindungsalternative sieht schließlich vor, dass das Abdeckelement nur mit dem Grundkörper verbunden ist und über dem Funktionselement lose liegt. Bei dieser Erfindungsalternative sind zwei quasi nebeneinander liegende Befestigungsbereiche oder-ebenen gegeben, nämlich zum einen die Befestigungsbereiche des Abdeckelements am Grundkörper, zum anderen der Befestigungsbereich des Funktionselements zum Grundkörper. Das Abdeckelement kann auch hier bevorzugt mit einem Kunststoffanteil ausgerüstet sein, um wiederum auch hier eine Kunststoffschweißverbindung zum Grundkörper zu realisieren, also eine stoffschlüssige Verbindung, wie sie auch zwischen dem Funktionselement und dem Grundkörper gegeben ist. Dies ermöglicht auch bei dieser dritten Erfindungsvariante die Befestigung aller Bauteile aneinander in einem einzigen Prozessschritt durch eine einmalige Energiezufuhr.

An dieser Stelle jedoch bereits der Hinweis, dass, soweit die Verbindung des Abdeckelements mit dem Grundkörper gegeben ist, anstelle einer stoffschlüssigen Schweißverbindung auch eine formschlüssige Verbindung gegeben sein kann, wie sie beispielsweise durch ein Annähen des Abdeckelements am Grundkörper unter Verwendung eines entsprechenden Nähfadens oder ein Vernieten erwirkt werden kann. Dies gilt für alle offenbarten Ausführungsbeispiele bzw. Ausführungsvarianten der Erfindung.

Das Abdeckelement selbst kann in Weiterbildung der Erfindung eine Durchbrechung aufweisen, beispielsweise einen länglichen Schlitz, wobei das Abdeckelement in diesem Fall beispielsweise nach Art eines rechteckigen, rahmenartigen Bauteils ausgeführt ist, das das im Bereich des Schlitzes angeordnete Funktionselement übergreift. Dabei kann die Ausgestaltung derart sein, dass das Funktionselement hinter der Durchbrechung verläuft. In diesem Fall ist beispielsweise das Funktionselement als flache Stabilisierungsstrebe ausgeführt, die auf dem Grundkörper aufgeschweißt ist und die von dem Abdeckelement, das beispielsweise am Grundkörper und am Funktionselement befestigt, bevorzugt angeschweißt ist, übergriffen ist. Durch den Schlitz ist das Funktionselement zu sehen, so dass einerseits erkannt werden kann, welcher Art das Funktionselement ist, andererseits kann dies auch zu Designgründen genutzt werden, beispielsweise wenn das Funktionselement eine sehr auffällige und von der Farbe des Grundkörpers abweichende Farbe aufweist oder Ähnliches.

Alternativ dazu kann das Abdeckelement auch bündig mit dem in die Durchbrechung greifenden Funktionselement verlaufen. In diesem Fall hat das Funktionselement beispielsweise einen T-förmigen Querschnitt, wobei es über den Querschenkel mit dem Grundkörper verschweißt ist und sich der vom Querschenkel erstreckende, relativ kurze Längsschenkel in die Durchbrechung des Abdeckelements erstreckt, mit der freien Abdeckelementseite aber im Wesentlichen bündig verläuft.

Schließlich kann in einer dritten Alternative das Funktionselement mit einer T-förmigen Querschnittsgestaltung auch die Durchbrechung durchgreifen, also wenige Millimeter oder weiter daraus hervorstehen. Das heißt, dass unterschiedliche Ausgestaltungen hinsichtlich der Geometrie des Funktionselements wie auch dessen geometrischer Anbindung bzw. seines Übergangs zum Abdeckelement gegeben sind.

Bevorzugt ist es ferner, wenn das Funktionselement an einer oder an mehreren Seiten einen seitlich vorstehenden Befestigungsabschnitt aufweist, der vom Abdeckelement übergriffen ist und über den es mit dem Grundkörper verbunden ist, wobei sich der an den Befestigungsabschnitt anschließende erhabene Abschnitt des Funktionselements in oder durch die Durchbrechung erstreckt. Wie bereits vorstehend beschrieben ist es denkbar, dass das Funktionselement einen T-förmigen Querschnitt aufweist. In diesem Fall sind zwei seitliche Befestigungsabschnitte vorgesehen, die vom Abdeckelement übergriffen sind. Denkbar ist aber auch ein L-förmiger Querschnitt des Funktionselements mit nur einem Befestigungsabschnitt, der vom Abdeckelement übergriffen ist. Der oder die Befestigungsabschnitte dienen der Verbindung des Funktionselements zum Grundkörper, wobei das Funktionselement aber auch in dem Bereich benachbart zu dem oder den Befestigungsabschnitten mit dem Grundkörper verschweißt sein kann, also über die gesamte Auflagefläche verschweißt sein kann.

Das Abdeckelement selbst kann mit dem Grundkörper und/oder dem Funktionselement bevorzugt stoffschlüssig verbunden sein, das heißt, dass auch hier eine Schweißverbindung bevorzugt erwirkt wird. Dies kann entweder dadurch erfolgen, dass das Abdeckelement selbst mit einem entsprechenden Kunststoffanteil, der aufgeschmolzen werden kann, ausgerüstet ist, beispielsweise mit einer aufkaschierten Kunststofffolie oder Ähnlichem, wie auch eine aufschmelzbare Zwischenlage vorgesehen werden kann, worauf nachfolgend noch eingegangen wird. Dies ist insbesondere zur Erwirkung einer stoffschlüssigen Verbindung zum Grundkörper zweckmäßig. Für eine stoffschlüssige Verbindung zum Funktionselement, das bereits aus Kunststoff besteht, ist eine entsprechende Kunststoffausrüstung des Abdeckelements nicht zwingend erforderlich, kann aber gleichermaßen vorgesehen sein. Alternativ oder gegebenenfalls auch kumulativ hierzu besteht aber auch die Möglichkeit, das Abdeckelement insbesondere mit dem Grundkörper auch formschlüssig zu verbinden, also beispielsweise zu vernähen, was ebenfalls zu einer festen Verbindung führt.

Eine vorteilhafte Weiterbildung der Erfindung sieht vor, dass das nur randseitig mit dem Grundkörper verbundene Abdeckelement an zumindest einer Seite unter Bildung einer zugänglichen Tasche offen ist. Bei dieser Ausgestaltung erstreckt sich das Abdeckelement seitlich gesehen relativ weit vom Funktionselement weg und ist in den Randbereichen, bis auf eine Seite oder einen entsprechenden Seitenabschnitt, fest mit dem Grundkörper verschweißt und/oder vernäht. Die Öffnung ermöglicht es, das Funktionselement, also beispielsweise ein Stabilisierungselement, in die Tasche einzuschieben und dann grundkörperseitig zu fixieren, wobei hierbei aber auch eine zusätzliche Fixierung des Abdeckelements am Funktionselement möglich ist, beispielsweise wiederum über eine simultan erwirkte stoffschlüssige Verbindung. Hier kann also beispielsweise das Abdeckelement zunächst am Grundkörper vernäht werden, wonach das Einschieben und Fixieren des Funktionselements durch Verschweißen erfolgt.

Wie bereits beschrieben ist es möglich, das Abdeckelement mit einer eine stoffschlüssige Verbindung erwirkenden, aufgeschmolzenen bzw. aufschmelzbaren Beschichtung zu versehen, die durch Energiezufuhr zur Erzeugung einer stoffschlüssigen Verbindung mit dem Grundkörper und/oder dem Funktionselement aufgeschmolzen ist. Das heißt, dass das Abdeckelement selbst mit einem entsprechenden Kunststoffanteil z.B. in Form eines Schmelzfadens ausgerüstet wird, der durch Energiezufuhr aufgeschmolzen werden kann, so dass die stoffschlüssige Klebeverbindung ermöglicht wird. Alternativ kann auch zwischen dem Abdeckelement und Grundkörper dem und/oder dem Funktionselement eine die stoffschlüssige Verbindung erwirkende, aufgeschmolzene Zwischenlage vorgesehen sein, also eine entsprechende Kunststofffolie oder Ähnliches, die beim Aufschmelzen sowohl eine stoffschlüssige Verbindung zum Abdeckelement an der einen Seite als auch zum Grundkörper oder dem Funktionselement an der anderen Seite erwirkt. Beide Ausgestaltungen, also einerseits die Kaschierung des Abdeckelements mit der Kunststofflage und andererseits die Zwischenschaltung der Zwischenlage, ermöglichen es, dass von der Außenseite des Textilteils nur die unbearbeitete Abdeckelementfläche zu sehen ist, das heißt, dass die Stoffschlussebene nicht sichtbar ist, da das Abdeckelement selbst eben nicht aufgeschmolzen wird, sondern nur seine entsprechende Kunststoffausrüstung oder die Zwischenlage.

Alternativ dazu ist es aber natürlich auch denkbar, dass das Abdeckelement selbst aus einem die stoffschlüssige Verbindung erwirkenden Material besteht oder ein solches enthält, das heißt, dass das Abdeckelement z.B. in Form einer Kunststofffolie selbst lokal zur Erwirkung des Stoffschlusses aufgeschmolzen wird.

Das Abdeckelement selbst ist bevorzugt ein flächiges Element, wozu sich insbesondere ein Textil eignet. Es ist bevorzugt natürlich undurchsichtig, da es als Abdeckelement ja das Funktionselement abschnittsweise und entsprechende stoffschlüssige Verbindungsbereiche abdecken soll. Vorzugsweise kann auch ein klettfähiges Textil verwendet werden, das es ermöglicht, an das Abdeckelement außenseitig noch weitere Elemente bei Bedarf fixieren zu können, oder das selbst eine Klettauflage aufweist, mit der es beispielsweise zur Erwirkung einer formschlüssigen Verbindung mit dem Grundkörper verbunden, also klettfixiert werden kann. Besonders bevorzugt ist dabei ein Velours respektive Klettvelours, bei dem es sich um eine mittelfeine Wirkware handelt, bei der Einzelfilamente von Fadenflottungen durch einen Ausrüstungsprozess wie Aufrauen insbesondere auf einer Walzen-Kratzen-Raumaschine zu Schlingen aufgestellt und verfestigt werden, um klettfähig zu sein. Die Erfindung ist jedoch nicht auf die Verwendung eines solchen Velours beschränkt, vielmehr kann jedes Textil respektive jede textile Schicht in Form eines Gewebes, Gewirkes oder Gestricks als Abdeckelement verwendet werden. Alternativ zur Verwendung eines Textils ist aber auch die Verwendung eines Abdeckelements in Form einer Kunststofffolie denkbar, wobei auch eine Kunststofffolie eine entsprechende Flexibilität aufweist, um die mitunter gegebenen Bewegungen des Grundkörpers oder des Funktionselements, mit dem das Kunststofffolie-Abdeckelement verbunden ist, nachzuvollziehen.

Der Grundkörper selbst ist zweckmäßigerweise ein Gestrickkörper, insbesondere ein kompressiver Gestrickkörper, der also durch Einstricken eines oder mehrerer elastischer Fäden, insbesondere Schussfäden eine definierte kompressive Eigenschaft aufweist, mithin also einen definierten Druck auf das übergriffene Gewebe respektive den übergriffenen Körperabschnitt erwirkt. Bezüglich der Ausgestaltung eines Textilteils mit kompressiven Eigenschaften sei beispielsweise auf die Definitionen in "Gütesicherung RAL-GZ387/1 Medizinische Kompressionsstrümpfe" und "Gütesicherung RAL-GZ387/2 Medizinische Kompressionsarmstrümpfe" der Gütezeichengemeinschaft medizinische Kompressionsstrümpfe e.V. verwiesen, wo entsprechende Güte- und Prüfbestimmungen für medizinische Kompressions(arm)strümpfe, die ebenfalls ein erfindungsgemäßes Textilteil sein können, angegeben sind.

Die stoffschlüssige Verbindung ist, wie ausgeführt, eine Kunststoffschweißverbindung. Diese kann durch Hochfrequenz- oder Ultraschallschweißen erwirkt werden, das heißt, dass durch Hochfrequenzrespektive Ultraschallschwingungen Energie in das aufzuschmelzende Material eingebracht wird, mithin also Wärme erzeugt wird, so dass das Kunststoffmaterial, sei es das Funktionselement selbst, sei es eine Kunststoffausrüstung des Abdeckelements, sei es eine Zwischenlage, zumindest lokal aufschmilzt und es zur Benetzung des benachbarten Bauteils und damit zu einer stoffschlüssigen Klebeverbindung nach Erkalten kommt.

Als Materialien führt das Funktionselement wie auch eine etwaige Kunststoffausrüstung des Abdeckelements oder die Kunststoff-Zwischenlage können unterschiedliche Kunststoffmaterialien verwendet werden, beispielsweise Polyurethan, Polyethylen, Polyvinylchlorid oder Polyamid, wobei beispielsweise das Funktionselement auch als 2- oder 3-Komponententeil ausgeführt sein kann. Die Aufzählung der verwendeten Kunststoffe ist nicht abschließend.

Dabei kann insbesondere das Funktionselement, aber auch das Abdeckelement, eine unterschiedliche Farbe wie der Grundkörper aufweisen. Dies kann einerseits Designgründen dienen, kann aber auch als Informationsangabe beispielsweise bezüglich der Steifigkeit eines als Stabilisierungselements dienenden Funktionselements dienen. Ist beispielsweise das Funktionselement in Form einer Stabilisierungsstrebe in grün ausgeführt, so handelt es sich um ein weicheres Stabilisierungselement als beispielsweise ein rot ausgeführtes Stabilisierungselement, während ein dunkelblau ausgeführtes Stabilisierungselement beispielsweise am härtesten ist.

Das Textilteil selbst kann unterschiedlichster Natur sein. Es kann sich um eine Bandage, eine Orthese oder einen Strumpf, insbesondere einen Kompressions(arm)strumpf handeln, wobei diese Aufzählung nicht abschließend ist. An der Bandage, der Orthese oder dem Strumpf kann ein Funktionselement unterschiedlicher Art respektive Geometrie angeordnet sein, beispielsweise ein längliches oder gebogenes stabartiges Funktionselement, ein gelenkartiges Funktionselement, ein als Gurtdurchführung ausgebildetes Funktionselement, ein als manuell greifbare Zughilfe, also als Anziehhilfe, ausgebildetes Funktionselement oder ein als Pelotte ausgebildetes Funktionselement. Es können also unterschiedlichste Funktionselemente verwendet werden, wobei auch diese Aufzahlung nicht abschließend ist. Je nach Art respektive Geometrie des Funktionselements gestaltet sich dann auch die Form respektive Geometrie des Abdeckelements und gegebenenfalls auch des Schlitzes, der am Abdeckelement vorgesehen ist.

Dabei ist es bevorzugt, das Funktionselement und damit auch das Abdeckelement an der Außenseite des textilen Grundkörpers anzuordnen. Gleichwohl ist es aber auch möglich, das Funktionselement und das Abdeckelement an der Innenseite des Grundkörpers zu platzieren.

Neben dem Textilteil selbst betrifft die Erfindung ferner ein Verfahren zur Herstellung eines solchen Textilteils, das sich dadurch auszeichnet, dass auf einem textilen Grundkörper wenigstens ein Funktionselement aus Kunststoff sowie wenigstens ein den Grundkörper sowie das Funktionselement nur abschnittsweise übergreifendes Abdeckelement angeordnet werden, wonach durch Energiezufuhr zwischen dem Funktionselement und dem Grundkörper eine stoffschlüssige Verbindung erwirkt wird.

Weiterhin kann vorgesehen sein, dass durch Energiezufuhr zwischen dem Funktionselement und dem Grundkörper sowie zwischen dem Abdeckelement und dem Funktionselement und/oder dem Grundkörper eine stoffschlüssige Verbindung erwirkt wird. Demgemäß wird also nicht nur zwischen dem Funktionselement und dem Grundkörper eine stoffschlüssige Verbindung erwirkt, sondern auch zur Befestigung des Abdeckelements am Funktionselement, am Grundkörper oder an beiden. Es finden also ausschließlich stoffschlüssige Verbindungen der Bauteile untereinander statt. Dies hat den besonderen Vorteil, dass sämtliche Befestigungen in einem einzigen Prozessschritt durch einmalige Energiezufuhr, beispielsweise in Form von Hochfrequenz- oder Ultraschallschwingungen im Rahmen eines Kunststoffschweißverfahrens, erzeugt werden können.

Des Weiteren kann vorgesehen sein, dass entweder das Funktionselement im Bereich des Übergriffs des Abdeckelements zur Erwirkung der Verbindung zum Abdeckelement sowie zum Grundkörper aufgeschmolzen wird, oder dass das Abdeckelement selbst oder eine am Abdeckelement vorgesehene Beschichtung oder eine zwischen das Abdeckelement und das Funktionselement und/oder den Grundkörper eingebrachte Zwischenlage einerseits sowie das Funktionselement andererseits zur Erwirkung der Verbindung des Abdeckelements zum Funktionselement und/oder zum Grundkörper und des Funktionselements zum Grundkörper aufgeschmolzen wird. Es sind also unterschiedliche Varianten möglich, die einzelnen Elemente miteinander zu verbinden, was insbesondere davon abhängt, ob das Abdeckelement selbst mit einem entsprechenden Kunststoffanteil, der zumindest teilweise aufschmelzbar ist und über den eine stoffschlüssige Verbindung zum Grundkörper/Funktionselement erwirkbar ist, versehen ist oder nicht.

Als Abdeckelement selbst wird bevorzugt ein Abdeckelement aus einem, vorzugsweise klettfähigen, Textil, insbesondere Velours verwendet, alternativ auch eine Kunststofffolie. Unabhängig davon ist das Abdeckelement bevorzugt flexibel, was im Falle eines Textils und einer entsprechend dünnen Kunststofffolie der Fall ist, und was es ermöglicht, dass das Abdeckelement entsprechende geometrische Verformungen des Grundkörpers respektive des Funktionselements nachvollzieht respektive mit ausführen kann.

Als Grundkörper wird bevorzugt ein solcher in Form eines Gestrickkörpers, insbesondere eines kompressiven Gestrickkörpers verwendet.

Schließlich wird bevorzugt ein Funktionselement sowie gegebenenfalls eine Beschichtung bzw. Ausrüstung des Abdeckelements oder eine Zwischenlage oder ein Abdeckelement selbst aus Polyurethan, Polyethylen, Polyvinylchlorid oder Polyamid verwendet.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Explosionsdarstellung eines erfindungsgemäßen Textilteils in einer Teilansicht, mit einem Grundkörper, einem Funktionselement und einem Abdeckelement vor deren Befestigung am Grundkörper,
- Fig. 2: die Anordnung aus Fig. 1 mit auf den Grundkörper aufgebrachtem Funktionselement und Abdeckelement während deren Befestigung,
- Fig. 3: die schweißverbundene Anordnung von Funktionselement und Abdeckelement am Grundkörper,
- Fig. 4: eine Teilansicht eines erfindungsgemäßen Textilteils einer zweiten Ausführungsform mit nur am Funktionselement befestigtem Abdeckelement,
- Fig. 5: eine Teilansicht eines erfindungsgemäßen Textilteils einer dritten Ausführungsform mit nur am Grundkörper befestigtem Abdeckelement,
- Fig. 6: eine Prinzipdarstellung eines erfindungsgemäßen Textilteils mit einem durch Vernähen formschlüssig am Grundkörper befestigten Abdeckelement und am Funktionselement verschweißten Abdeckelement,
- Fig. 7: eine Prinzipdarstellung eines erfindungsgemäßen Textilteils in Form einer Bandage mit einem stabförmigen Funktionselement,
- Fig. 8: eine Prinzipdarstellung eines erfindungsgemäßen Textilteils in Form einer Orthese mit einem Funktionselement in Form eines Gelenks und
- Fig. 9: eine Prinzipdarstellung eines erfindungsgemäßen Textilteils in Form eines Strumpfes mit einem als manuell zu greifendem Griff, der als Anziehhilfe dient, ausgeführten Funktionselement.

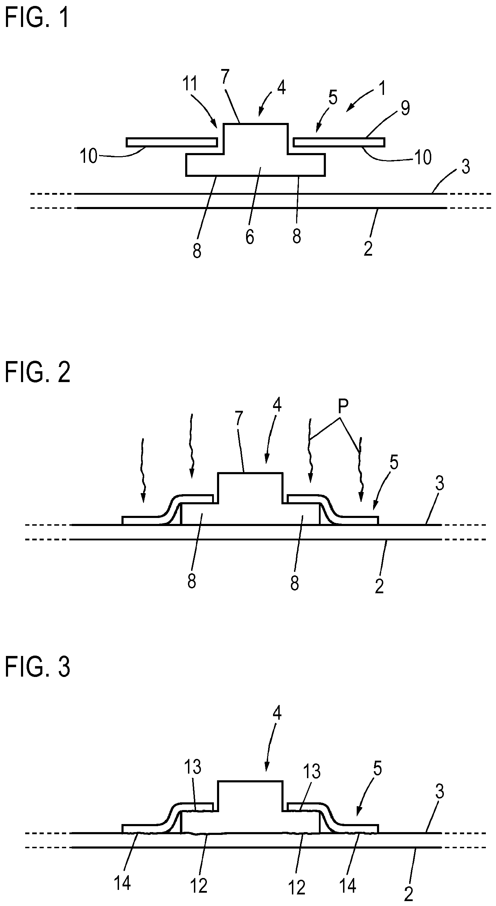

Fig. 1 zeigt eine Explosionsansicht eines erfindungsgemäßen Textilteils 1, umfassend einen textilen Grundkörper 2 in Form eines Gestrickkörpers, insbesondere eines kompressiven Gestrickkörpers. Auf dessen Außenseite 3 soll eine Kombination aus einem Funktionselement 4 und einem Abdeckelement 5 aufgebracht und daran befestigt werden, wobei im beschriebenen Ausführungsbeispiel einerseits eine stoffschlüssige Verbindung des Funktionselements 4 zur Außenseite 3 des Grundkörpers erfolgen soll, andererseits aber auch eine stoffschlüssige Verbindung des Abdeckelements 5 einerseits zum Funktionselement 4, andererseits auch zur Außenseite 3 des Grundkörpers.

Zu diesem Zweck werden, siehe Fig. 2, das Funktionselement 4 und das Abdeckelement 5 auf die Außenseite 3 des Grundkörpers 2 aufgesetzt. Im gezeigten Ausführungsbeispiel ist das Funktionselement T-förmig, es weist also einen breiten Querschenkel 6 sowie einen davon abstehenden Längsschenkel 7 auf. Mit dem Querschenkel 6 liegt das Funktionselement 4 auf der Außenseite 3 auf. Die seitlichen, vom Längsschenkel 7 abstehenden Befestigungsabschnitte 8 dienen der Verbindung des Funktionselements 4 einerseits zum Grundkörper 2, andererseits aber auch an der anderen Seite der Verbindung des Abdeckelements 5 mit dem Funktionselement 4. Das Abdeckelement 5 ist bevorzugt ein flexibles Element, insbesondere ein Textil, vorzugsweise ein Velours. Es weist also einen Textilabschnitt 9 auf, an dessen Unterseite im gezeigten Beispiel eine Kunststoffbeschichtung 10 aufkaschiert ist, das heißt, dass das Abdeckelement 5 mit einem Kunststoffanteil ausgerüstet ist, der, worauf nachfolgend noch eingegangen wird, aufgeschmolzen werden kann.

Anstelle einer fest an dem Textilkörper 9 aufkaschierten Beschichtung 10 kann auch eine separate, über eine Kunststofffolie gebildete Zwischenlage vorgesehen, das heißt, dass es sich bei dem in Fig. 1 mit dem Bezugszeichen 10 gezeigten Bauteil auch um eine separate, nicht fest mit dem Abdeckelement 5 verbundene Kunststoff-Zwischenlage handeln kann, die gleichermaßen aufgeschmolzen werden kann und die entsprechenden Verbindungen erwirken kann, wie im nachfolgenden beschrieben. Daneben kann das Abdeckelement 5 natürlich auch selbst in Form einer Kunststofffolie, die aufgeschmolzen werden kann, ausgeführt sein.

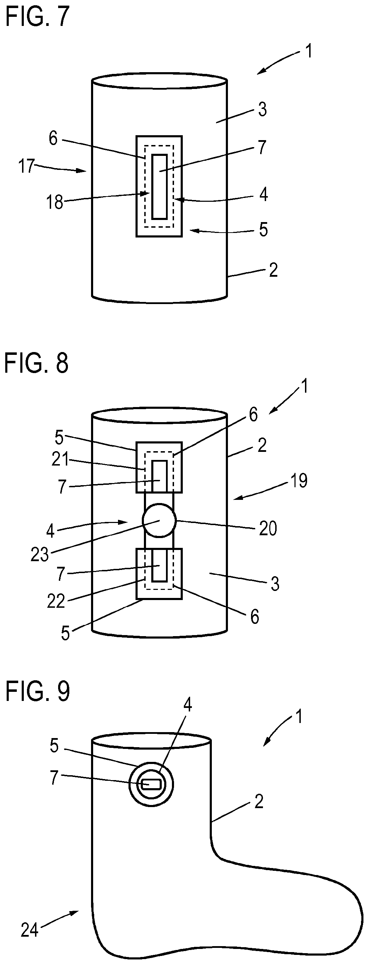

Das Abdeckelement 5 selbst ist quasi rechteckig-länglich ausgeführt und weist einen mittigen, länglichen Schlitz 11 auf, der, siehe Fig. 2, vom Längsschenkel 7 durchgriffen ist, dieser ragt also etwas aus dem Abdeckelement 5 heraus. Aufgrund seiner rechteckigen Ausgestaltung (siehe hierzu auch Fig. 7) rahmt das Abdeckelement 5 den vorspringenden Längssteg 7 allseitig ein.

Die Verbindung von Funktionselement 4 und Abdeckelement 5 untereinander sowie mit dem Gestrickteil 2 erfolgt durch Energiezufuhr, indem Strahlungsenergie, wie durch die Pfeile P in Fig. 2 dargestellt ist, in Form von Hochfrequenz- oder Ultraschallstrahlung appliziert wird. Das heißt, es findet ein Hochfrequenz- oder Ultraschallverschweißen statt. Dies führt dazu, dass einerseits das Funktionselement 4 im Bereich seiner Befestigungsabschnitte 8 beidseits aufschmilzt, wie auch die Kunststoffbeschichtung 10 des Abdeckelements 5 aufschmilzt. Es kommt zu einem Benetzen einerseits der aufgeschmolzenen Kunststoffmassen am Funktionselement 4 und am Abdeckelement 5, die sich stoffschlüssig verbinden, und andererseits auch zum Benetzen des Gestrickkörpers 2 an seiner Außenseite durch die aufgeschmolzene Kunststoffbeschichtung 10. Nach Abkühlen ergeben sich entsprechende stoffschlüssige Verbindungen, wie in Fig. 3 durch die gewellten Linien angedeutet ist, nämlich einerseits erste stoffschlüssige Verbindungen 12 zwischen dem Funktionselement 4 und dem Grundkörper 2 an dessen Außenseite 3, zum anderen stoffschlüssige Verbindungen 13 zwischen dem Funktionselement 4 und dem Abdeckelement 5 im Bereich seines Übergriffs über die Befestigungsabschnitte 8, und schließlich stoffschlüssige Verbindungen 14 zwischen dem Abdeckelement 5 und dem Grundkörper 2 an seiner Außenseite 3.

Wie ersichtlich ist, werden sämtliche stoffschlüssigen Verbindungen 12, 13, 14 in einem einzigen Prozessschritt durch eine einmalige Energiezufuhr ausgebildet, das heißt, dass alle Elemente untereinander in einem einzigen Prozessschritt miteinander verbunden werden können.

Das Funktionselement 4 selbst ist aus Kunststoff, bevorzugt aus Polyurethan, Polyethylen, Polyvinylchlorid oder Polyamid, wobei diese Aufzählung nicht abschließend ist. Gleiche Materialien können auch für die aufkaschierte Beschichtung 10 des Abdeckelements 5 verwendet werden.

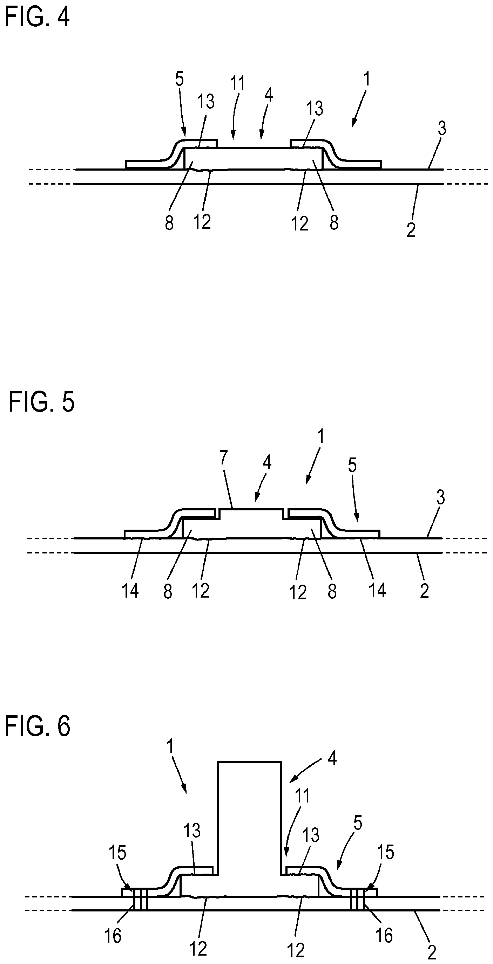

Fig. 4 zeigt ein Ausführungsbeispiel eines erfindungsgemäßen Gestrickteils 1 mit daran befestigtem Funktionselement 4 und Abdeckelement 5. Die hier erwirkten stoffschlüssigen Verbindungen sind ebenfalls durch Hochfrequenz- oder Ultraschallschweißen erzeugt. Realisiert sind einerseits die stoffschlüssigen Verbindungen 12 des Funktionselements 4 zum Grundkörper 2, also dem Gestrickkörper. Daneben sind stoffschlüssige Verbindungen 13 zwischen dem Funktionselement 4 und dem Abdeckelement 5 im Bereich der Befestigungsabschnitte 8 des Funktionselements 4 realisiert. Über dem Grundkörper 2 liegt hier das Funktionselement 4 lose auf, das heißt, dass das Abdeckelement 5 und Grundkörper 2 miteinander nicht fest verbunden sind.

Bei dieser Erfindungsausgestaltung muss das Abdeckelement 5 nicht mit einer Beschichtung 10 ausgerüstet sein, da allein das lokale Aufschmelzen des Funktionselements 4 im Bereich der Befestigungsabschnitte 8 ausreichend ist, um beide stoffschlüssig miteinander zu verbinden.

Wie Fig. 4 zeigt, ist hier das Funktionselement 4 als quasi I-förmige, also ebene Stabilisierungsstrebe ausgebildet, die unterhalb der Durchbrechung 11 liegt, also nicht in diese respektive durch diese greift.

Eine weitere Erfindungsausgestaltung eines Textilteils 1 zeigt Fig. 5. Vorgesehen ist wiederum das Funktionselement 4, das Abdeckelement 5 sowie der Grundkörper 2, an dem das Funktionselement 4 wiederum stoffschlüssig über die Verbindungen 12 schmelzbefestigt ist. Bei dieser Ausgestaltung ist das Abdeckelement 5 wiederum mit einer entsprechenden Kunststoffausrüstung beispielsweise in Form der Beschichtung 10 versehen, die es ermöglicht, dass die stoffschlüssigen Schmelzverbindungen 14 zwischen dem Abdeckelement 5 und dem Grundkörper 2 erwirkt werden. Am Funktionselement 4 respektive auf den Befestigungsabschnitten 8 jedoch liegt das Abdeckelement 5 lose auf, hier ist also keine Verbindung vorgesehen. Bei dieser Erfindungsausgestaltung ist also das Abdeckelement 5 beispielsweise nur randseitig mit der Beschichtung 10 versehen, nicht aber im Bereich des Überlapps zum Funktionselement 4 hin.

Wiederum zeigt das Funktionselement 4 eine etwas andere Querschnittsgeometrie. Es weist zwar auch hier einen Längsschenkel 7 auf, dieser ist jedoch sehr kurz, so dass er letztlich bündig mit dem Abdeckelement 5 verläuft.

Eine vierte Erfindungsvariante eines erfindungsgemäßen Textilteils 1 zeigt Fig. 6. Vorgesehen ist wiederum das Funktionselement 4 sowie das Abdeckelement 5 und der Grundkörper 2. Das Kunststoff-Funktionselement 4 ist hier wiederum durch Hochfrequenz- oder Ultraschallschweißen mit dem Grundkörper 2 über die stoffschlüssigen Verbindungen 12 verbunden. Das Abdeckelement 5 ist mit dem Funktionselement 4 ebenfalls über stoffschlüssige Verbindungen 13 verbunden, wobei diese durch lokales Aufschmelzen der Befestigungsabschnitte 8 an deren Oberfläche erwirkt werden, das heißt, dass das Abdeckelement 5 nicht mit einer entsprechenden Kunststoffbeschichtung 10 ausgerüstet ist. Es sind also hier zwei quasi übereinander liegende stoffschlüssige Verbindungen 12, 13 realisiert.

Das Abdeckelement 5 ist des Weiteren randseitig mit dem Grundkörper 2 verbunden, jedoch über eine formschlüssige Verbindung 15, die hier durch Vernähen mit einem Nähfaden 16 erwirkt sind. Wiederum sind hier also alle drei Elemente untereinander fest verbunden.

Bei dieser Erfindungsausgestaltung erstreckt sich das Funktionselement 4 mit einem relativ langen Längsschenkel 7 weit aus der Durchbrechung 11 des Abdeckelements 5 heraus. Der Längsschenkel 7 kann beispielsweise mit einer Durchbrechung versehen sein, so dass er quasi griffartig ausgeführt ist und manuell gegriffen werden und als Anziehhilfe dienen kann, wozu er beispielsweise an einem Strumpf oder Ähnlichem, der das Grundgestrick bildet, befestigt ist.

Fig. 7 zeigt eine Prinzipdarstellung einer ersten konkreten Ausgestaltung eines Textilteils 1 hier in Form einer Bandage 17. Gezeigt ist der Grundkörper 2, der hier, wie bei einer Bandage üblich, schlauchförmig ist. An der Außenseite 3 des Grundkörpers ist einerseits das Funktionselement 4 befestigt, andererseits ist dort auch das Abdeckelement 5 angeordnet. Das Funktionselement 4 ist hier als längliche Stabilisierungsstrebe 18 ausgeführt, die im Querschnitt beispielsweise T-förmig ist, wie in den Fig. 1 - 3 dargestellt ist. Gestrichelt ist einerseits der Querschenkel 6 gezeigt, während der Längsschenkel 7, der aus der Durchbrechung 11 des Abdeckelements 5 hervortritt, ebenfalls dargestellt ist. Für diese Ausgestaltung sind alle vorstehend beschriebenen Verbindungsvarianten denkbar.

Fig. 8 zeigt eine zweite konkrete Erfindungsausgestaltung eines erfindungsgemäßen Textilteils 1 hier in Form einer Orthese 19, wiederum umfassend einen Grundkörper 2, an dessen Außenseite 3 das Funktionselement 4 hier in Form eines Gelenks 20 angeordnet ist. Das Gelenk 20 weist zwei Schenkel 21, 22 auf, die über eine Gelenkverbindung 23 miteinander verbunden sind. Jeder Schenkel 21, 22 ist von einem separaten Abdeckelement 5 abschnittsweise übergriffen. Wiederum weist auch hier jeder Gelenkschenkel 21, 22 einen entsprechenden Querschenkel 6, hier gestrichelt gezeigt, sowie einen Längsschenkel 7, der die entsprechende abdeckelementseitige Durchbrechung 11 durchgreift, auf, während die Gelenkverbindung 23 freiliegt. Auch hier sind wiederum sämtliche vorstehend beschriebenen, unterschiedlichen Befestigungsvarianten der Elemente aneinander denkbar.

Bei dieser Erfindungsausgestaltung besteht beispielsweise auch die Möglichkeit, ausgehend von der Befestigungsvariante gemäß Fig. 6, die beiden Abdeckelemente 5 zunächst über die formschlüssige Verbindung 16 am Grundkörper 2 anzunähen, die Naht aber nur um drei Seiten zu ziehen, so dass an einer Seite die einander zuweisenden Elementseiten nicht vernäht, sondern offen sind, so dass die Gelenkschenkel 21, 22 hier in diese taschenförmigen Abdeckelemente 5 eingeschoben werden können, wonach erst die Schweißverbindung zwischen Funktionselement 4 und Grundkörper 2 sowie zwischen Abdeckelement 5 und Funktionselement 4 erwirkt werden kann.

Schließlich zeigt Fig. 9 eine dritte konkrete Erfindungsvariante eines Textilkörpers 1, mit einem Grundkörper 2 in Form eines Strumpfes 24. Im Bereich des oberen Strumpfes 24 ist hier wiederum das Funktionselement 4 und das Abdeckelement 5 angeordnet, wobei hier das Funktionselement 4 quasi rundlich-scheibenförmig ist und einen relativ weit vorspringenden Längsschenkel 7 aufweist, der die entsprechende Durchbrechung im Abdeckelement 5, das auch hier rundlich ausgeführt ist, durchgreift. Hierbei handelt es sich beispielsweise um einen Griff, wie er bereits zu Fig. 6 beschrieben wurde. Auch hier sind die entsprechenden Befestigungsvarianten denkbar, wobei insbesondere Varianten, bei denen das Abdeckelement 5 sowohl mit dem Funktionselement 4 als auch dem Getrickkörper 2 verbunden ist, aufgrund der beim Hochziehen wirkenden Kräfte bevorzugt sind.

## Patentansprüche

1. Textilteil, insbesondere textiles orthopädisches Hilfsmittel, umfassend einen textilen Grundkörper (2) sowie wenigstens ein am Grundkörper (2) angeordnetes Funktionselement (4) aus Kunststoff, **dadurch gekennzeichnet, dass** wenigstens ein das auf dem Grundkörper (2) angeordnete Funktionselement (4) nur abschnittsweise übergreifendes, flächiges Abdeckelement (5) vorgesehen ist, das sich auch über den Grundkörper (2) erstreckt, wobei das Abdeckelement (5) mit dem Grundkörper (2) und/oder dem Funktionselement (4) verbunden ist und das Funktionselement (4) mit dem Grundkörper (2) stoffschlüssig verbunden ist.

2. Textilteil nach Anspruch 1, **dadurch gekennzeichnet, dass** das Abdeckelement (5) eine Durchbrechung (11) aufweist, wobei das Funktionselement (4) hinter der Durchbrechung (11) verläuft, oder wobei das Abdeckelement (5) bündig mit dem in die Durchbrechung (11) greifenden Funktionselement (4) verläuft, oder wobei das Funktionselement (4) die Durchbrechung (11) durchgreift.

3. Textilteil nach Anspruch 2, **dadurch gekennzeichnet, dass** das Funktionselement (4) an einer oder mehreren Seiten einen seitlich vorstehenden Befestigungsabschnitt (8) aufweist, der vom Abdeckelement (5) übergriffen ist und über den es mit dem Grundkörper (2) verbunden ist, wobei der sich an den Befestigungsabschnitt (8) anschließende erhabene Abschnitt (7) des Funktionselements (4) in oder durch die Durchbrechung (11) erstreckt.

4. Textilteil nach einem der vorangehenden Ansprüche, **dadurch gekennzeichnet, dass** das Abdeckelement (5) mit dem Grundkörper (2) und/oder dem Funktionselement (4) stoffschlüssig und/oder formschlüssig verbunden ist.

5. Textilteil nach einem der vorangehenden Ansprüche, **dadurch gekennzeichnet, dass** das nur randseitig mit dem Grundkörper (2) verbundene Abdeckelement (5) an zumindest einer Seite unter Bildung einer zugänglichen Tasche offen ist.

6. Textilteil nach einem der vorangehenden Ansprüche, **dadurch gekennzeichnet, dass** das Abdeckelement (5) eine die stoffschlüssige Verbindung erwirkende, aufgeschmolzene Beschichtung (11) aufweist, oder dass zwischen dem Abdeckelement (5) und dem Funktionselement (4) und/oder dem Grundkörper (2) eine die stoffschlüssige Verbindung erwirkende, aufgeschmolzene Zwischenlage vorgesehen ist.

7. Textilteil nach einem der Ansprüche 1 bis 5, **dadurch gekennzeichnet, dass** das Abdeckelement (5) selbst aus einem die stoffschlüssige Verbindung erwirkenden Material besteht oder ein solches enthält.

8. Textilteil nach einem der vorangehenden Ansprüche, **dadurch gekennzeichnet, dass** das Abdeckelement (5) ein, vorzugsweise klettfähiges, Textil, insbesondere Velours oder eine Kunststofffolie ist. vorzugsweise beschichtet

9. Textilteil nach einem der vorangehenden Ansprüche, **dadurch gekennzeichnet, dass** der Grundkörper (2) ein, insbesondere kompressiver, Gestrickkörper ist.

10. Textilteil nach einem der vorangehenden Ansprüche, **dadurch gekennzeichnet, dass** das Funktionselement (4) sowie gegebenenfalls die Beschichtung (10) oder die Zwischenlage oder das Abdeckelement (5) aus Polyurethan, Polyethylen, Polyvinylchlorid oder Polyamid ist.

11. Textilteil nach einem der vorangehenden Ansprüche, **dadurch gekennzeichnet, dass** es eine Bandage (17), eine Orthese (19) oder ein Strumpf (24), insbesondere ein Kompressionsstrumpf ist, an der oder dem ein längliches oder gebogenes stabartiges Funktionselement (4, 18), ein gelenkartiges Funktionselement (4, 20), ein als Gurtdurchführung ausgebildetes Funktionselement, ein als manuell greifbare Zughilfe ausgebildetes Funktionselement (4) oder ein als Pelotte ausgebildetes Funktionselement vorgesehen ist.

12. Verfahren zur Herstellung eines Textilteils nach einem der vorangehenden Ansprüche, **dadurch gekennzeichnet, dass** auf einem textilen Grundkörper (2) wenigstens ein Funktionselement (4) aus Kunststoff sowie wenigstens ein den Grundkörper (2) sowie das Funktionselement (4) nur abschnittsweise übergreifendes Abdeckelement (5) angeordnet werden, wonach durch Energiezufuhr zwischen dem Funktionselement (4) und dem Grundkörper (2) eine stoffschlüssige Verbindung (12) erwirkt wird.

13. Verfahren nach Anspruch 12, **dadurch gekennzeichnet, dass** durch Energiezufuhr zwischen dem Funktionselement (4) und dem Grundkörper (2) sowie zwischen dem Abdeckelement (5) und dem Funktionselement (4) und/oder dem Grundkörper (2) eine stoffschlüssige Verbindung (12, 13, 14) erwirkt wird.

14. Verfahren nach Anspruch 13, **dadurch gekennzeichnet, dass** entweder das Funktionselement (4) im Bereich des Übergriffs des Abdeckelements (5) zur Erwirkung der Verbindung zum Abdeckelement (5) sowie zum Grundkörper (2) aufgeschmolzen wird, oder dass das Abdeckelement (5) selbst oder eine am Abdeckelement (5) vorgesehene Beschichtung (10) oder eine zwischen das Abdeckelement (5) und das Funktionselement (4) und/oder den Grundkörper (2) eingebrachte Zwischenlage einerseits sowie das Funktionselement (4) andererseits zur Erwirkung der Verbindung des Abdeckelements (5) zum Funktionselement (4) und/oder zum Grundkörper (2) und des Funktionselements (4) zum Grundkörper (5) aufgeschmolzen wird.

15. Verfahren nach einem der Ansprüche 12 bis 14, **dadurch gekennzeichnet, dass** ein Abdeckelement (5) aus einem, vorzugsweise klettfähigen, Textil, insbesondere Velours oder aus einer Kunststofffolie verwendet wird, und/oder dass ein Grundkörper (2) in Form eines, insbesondere kompressiven, Gestrickkörpers, verwendet wird, und/oder dass ein Funktionselement (4) sowie gegebenenfalls eine Beschichtung (10) des Abdeckelements (5) der eine Zwischenlage oder ein Abdeckelement (5) selbst aus Polyurethan, Polyethylen, Polyvinylchlorid oder Polyamid verwendet wird.
